# EUROPEAN PATENT SPECIFICATION

(11) **EP 2 410 922 B1**
(45) Date of publication and mention of the grant of the patent: **02.10.2013**
(21) Application number: 10709410.4
(22) Date of filing: 10.03.2010
(51) Int. Cl.: A61B 8/12

(54) **APPARATUS FOR CARRYING OUT DIAGNOSTIC AND/OR THERAPEUTIC PROCEDURES**
GERÄT ZUR DURCHFÜHRUNG VON DIAGNOSTISCHEN UND/ODER THERAPEUTISCHEN VERFAHREN
APPAREIL PERMETTANT DE REALISER DES PROCEDURES DIAGNOSTIQUES ET/OU THERAPEUTIQUES

(30) Priority: 11.03.2009 GB 0904194
(43) Date of publication of application: 01.02.2012
(73) Proprietor: Lares Technologies Limited, Stratford upon Avon Warwickshire CV37 6JG (GB)
(72) Inventor: McENEANEY, David, County Armagh BT63 5QQ (GB)
(74) Representative: Parnham, Kevin
(86) International application number: PCT/EP2010/001479
(87) International publication number: WO 2010/102794

(56) References cited:
- EP-A1- 1 859 732
- US-A- 5 699 805
- US-A1- 2007 083 100

## Description

This invention relates to an apparatus for carrying out diagnostic and/or therapeutic procedures and in particular to an apparatus procedures and/or diagnosis comprising a hollow catheter for insertion into the body, an in particular to such an apparatus incorporating an imaging device adapted to provide an image of the tissue ahead of and lateral to the catheter tip.

The intrinsic rhythm of the heart is generated in the sino-atrial node, at the junction of the right atrium and superior vena cava. A wave of activation passes over the upper heart chambers (atria), then is conveyed to the lower chambers of the heart (ventricles) by way of the atrio-ventricular node, His bundle and bundle branch/Purkinje fibres. This permits coordinated contraction of the atria and ventricles. If the electrical system of the heart is damaged dangerously slow or fast heart rhythms may ensue (bradyarrhythmias or tachyarrhythmias respectively). In many cases these abnormal heart rhythms may result in significant symptoms including dizzy spells or blackouts. In other circumstances arrhythmias may be fatal. By inserting electrodes into one or more of the chambers of the heart, via a large vein, it is possible to correct slow heart rhythms by delivering precisely controlled electrical impulses to the appropriate heart chamber. These impulses are generated by a battery and circuitry contained within a pacemaker generator. Power leads link the electrodes to a small hermetically sealed metal box, called the generator, that contains the battery and electronics. Implantable cardiovascular defibrillators have the added ability to deliver higher energy electrical shocks capable of correcting dangerous fast heart rhythms (tachycardias)..

750,000 pacemakers and implantable cardiovascular defibrillators are inserted annually in Europe and 850,000 implantable devices are inserted annually in the United States.

As these devices increase in complexity, a greater number of implanted leads may be required, up to three in some cases. However, such cardiac leads may be prone to failure due to the mechanical stresses caused by movement or impact or they may require removal due to infection. Typically 4% to 7% of leads may require removal over the lifetime of a patient due to infection or malfunction, and studies have shown that up to 20% of cardiac leads may require replacement within ten years. Cardiac leads typically terminate in a tined tip or screw for anchoring the lead into the muscle tissue of the heart (myocardium). Removal of the lead can result in damage to the heart, particularly where fibrosis has occurred around the tip of the lead. Furthermore, while the leads are formed from materials chosen to be as inert as possible, tissue tends to grow over the leads, particularly where they are in contact with the walls of the veins through which they pass, leading to adhesion of the lead to the vessel

The removal of pacemaker/ICD leads is typically achieved by advancing a sheath or catheter over the lead, the forward tip of the catheter cutting through adhesions of the tissue to the lead. The lead can then be pulled back through the catheter and removed from the body, the catheter acting as a guide for removal of the lead. The cutting action of the catheter can be enhanced by providing a concentric second sheath around the catheter that can advanced relative to the catheter. In other versions, optical fibres can be provided within the wall of the sheath by means of which laser energy can be used to cut through the tissue ahead of the catheter tip. In another version, bipolar electrocautery electrodes are incorporated into the tip of the catheter for separating tissue ahead of the catheter tip using radiofrequency energy.

Many other intravascular and keyhole surgeries and diagnostic techniques involve the insertion of hollow catheters into the body and, in particular, into the venous and arterial systems.

A major problem with existing lead removal technology, and other techniques involving the insertion into the body of hollow catheters, is that the catheter is typically inserted under X- ray control, with no method of directly visualising the point of contact of the catheter with tissue immediately ahead of or adjacent to the catheter tip, leading to the risk of damage to tissue and the possible tearing and rupturing of the walls of blood vessels or the heart chambers. External imaging techniques are able to visualise dense structures within the body, such as bones (X-ray fluoroscopy). However, such techniques do not permit visualisation of the point of contact of the catheter tip with adjacent soft tissues. The pacemaker lead and catheter may thus be imaged using x-rays but the vessels and heart are not visible using this approach, thus the relationship of fibrosis and adhesions to the lead cannot be established.

Intravascular ultrasound imaging has been used in the past to provide a cross sectional image of a coronary artery by advancing a small imaging transducer (<1mm diameter and length), placed at the end of a rotating driveshaft containing the signal cables, through a catheter and then slowly retracting the probe to provide an image of the cross section of the probe along the length of the artery. There are various sizes and frequency spectrum characteristics of probes. The usual probe for coronary arteries uses 10-20Mhz. For coronary arteries the probe resides within a thin polythene tube (through which the sound waves can be transmitted and received by the transducer). The probe can move longitudinally in the tube thus allowing sequential cross sections of the vessel to be obtained. An example of such known ultrasonic imaging probe is the Atlantis SR pro imaging catheter produced by Boston Scientific.

However, such known ultrasonic probes are only able to produce cross sectional images of the artery and cannot provide a forward looking image of the leading tip of a catheter. Furthermore, such probes are advanced and retracted coaxially through the centre of a guide catheter, therefore the catheter cannot serve a further purpose, such as cardiac lead removal.

According to the present invention there is provided an apparatus as defined in claim 1, partitioned relative to the document EP 1859732A1, and further aspects as defined in dependent claims these to.

An embodiment of the present invention will now be described, by way of example only, with reference to the accompanying drawings, in which:-
Figure 1 is a longitudinal sectional view through a cardiac lead removal apparatus according to an embodiment of the present invention; and

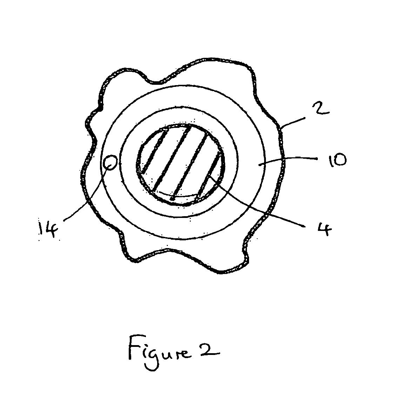

Figure 2 is a sectional view on line A-A of Figure 1.

As shown in the drawings, a cardiac lead removal apparatus according to an embodiment of the present invention comprises a sheath or catheter 10 adapted to be insertable into a vein 2 with the catheter concentrically arranged around a cardiac lead 4 whereby the catheter 10 can be advanced along the lead 4 such that the tip 12 of the catheter 10 effectively cuts through and separates the lead from surrounding tissue and provides a guide tube for removal of the cardiac lead 4 from the body.

In order to provide an image of the point of contact of the catheter with the tissue through which it is passed, an ultrasonic imaging device is incorporated into the catheter. A polythene guide tube 14 is located within the wall of the catheter 10, within which is located a thin elongate cable (not shown) having one or more ultrasonic transducers located at a distal end thereof, leads for the or each transducer extending through the cable. Alternatively other suitable imaging devices, such as an optical coherence tomography device, may be used.

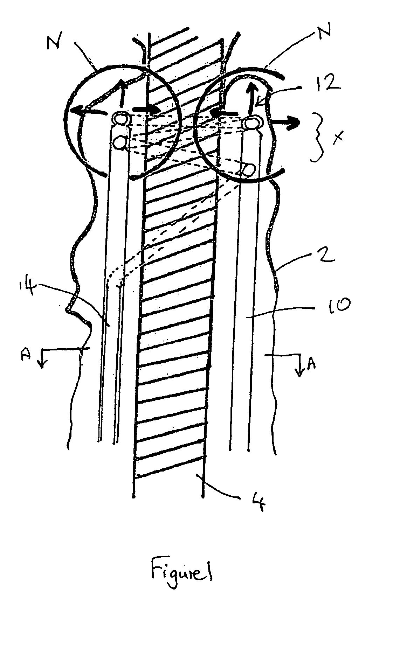

The elongate cable is arranged to be rotatable and axially translatable within the guide tube 14. The guide tube 14 runs longitudinally of the catheter 10, except for a distal portion X of the guide tube 14 at or adjacent the tip of the catheter 10, which extends in a substantially helical or circumferential configuration around a distal region of the catheter 10 whereby the ultrasonic transducer can be reciprocated around the circumference of the tip of the catheter, and optionally also rotated, to produce a toroidal image of the tissue immediately around and ahead of the catheter tip, as shown in Figure 1 (see region marked N), providing forward, medial and lateral imaging around the circumference of the catheter tip, visualising adjacent vascular structures and pacemaker/defibrillator lead.

The guide tube 14, and the ultrasonic imaging device located therein, are located entirely within the wall of the catheter 10 such that the inner and outer surfaces of the catheter 10 remain smooth, avoiding snagging on the walls of the vein 2 through which the catheter 10 is passed and preventing the cardiac lead 4 from snagging on the inner wall of the catheter 10.

To image anterior and laterally to the tip of the catheter 10 requires the ultrasonic imaging device to be positioned perpendicular to the axis of the catheter and move circumferentially around (but within) it.

The image generated by the ultrasonic imaging device will be approximately toroidal so some processing will be needed to appropriately display this (e.g. in different cross sections or ideally 3D). Such processing can be provided by a computer connected to the ultrasonic imaging device.

IVUS (intravascular ultrasound) transducer technology is already available in various specifications which would need to be tailored to the device. IVUS as currently used in small arteries projects the ultrasound and receives the reflected images of arteries through a very thin plastic sheath - the transducer would thus can be contained within such a sheath at the catheter tip, so the rotation would not be evident viewed from outside the extraction sheath/catheter. Suitable computer software will be required to reconstruct a 3D image of the end of sheath, adjacent vessel and pacemaker lead. It will be possible to orient the imaging plane using the varying relationship to the helical channel to the catheter tip as a marker (x-y etc).

The present invention permits other extraction apparatus to be used as a second sheath, either internal to or external to the catheter 10. If internal, the second sheath would lie between the pacemaker lead and the catheter 10.

Current lead extraction technology, mechanical, electrocautery or laser, is compatible with the present invention.

While the embodiment of the present invention described above has been described in relation to a cardiac lead removal device, such can be equally applied to any other medical application wherein it is required to insert a hollow catheter into a body, the invention providing imaging of the point of contact of the catheter with body tissue, avoiding the prior art problems inherent with blind insertion of catheters, particularly in relation to intravascular catheters. The helical arrangement of the guide tube at the catheter tip thus provides a series of 2 dimensional cross sectional views around the circumference of the catheter tip, and with suitable image manipulation, a 3 dimensional reconstruction of the tip of the catheter and adjacent structures as it is advanced through the body.

Additional lumens or catheters may be provided within or alongside the catheter 10, such additional lumens or catheters housing other diagnostic and/or therapeutic devices, such as guide wires, stents, balloons, biopsy forceps, electrode catheters, such as mapping and/or ablation catheters or any other similar device, wherein the image generated by the imaging device may facilitate the use of such devices and the accurate positioning of such devices within the body. Such additional devices may be used for tissue sampling, in the case of biopsy forceps, the diagnosis and treatment of cardiac arrhythmias.

The invention is not limited to the embodiment(s) described herein but can be amended or modified without departing from the scope of the present invention.

## Claims

1. An apparatus for carrying out diagnostic and/or therapeutic procedures, said apparatus comprising a hollow tubular catheter (10) for insertion into the venous or arterial system (2) or other body cavity of a patient, said catheter having a tip (12), an imaging device being associated with the tip of the catheter, said imaging device being adapted to provide an image of the tissue ahead of and lateral to the catheter tip, wherein the imaging device is provided at a distal end of an elongate guide member guided within a guide passage (14) to be axially translatable within the guide passage, **characterized in that** a portion (x) of the guide passage at or adjacent the tip of the catheter extending in a circumferential configuration around a distal region of the catheter perpendicular to the Major axis of the catheter whereby the imaging device can be at least partially reciprocated circumferentially around part of the tip of the catheter to produce a toroidal or substantially toroidal image (N) of the tissue immediately around and ahead of the catheter tip.

2. An apparatus as claimed in claim 1, wherein the imaging device is adapted to provide forward and lateral visualisation of tissue adjacent the catheter tip.

3. An apparatus as claimed in claim 1 or claim 2, wherein said imaging device is adapted to distinguish between muscle, bone and blood and thus determine if the catheter is being advanced into areas where damage to tissue, such as the walls of veins and arteries, may occur.

4. An apparatus as claimed in any preceding claim, wherein said imaging device comprises an ultrasonic imaging device comprising one or more ultrasonic transducers.

5. An apparatus as claimed in any preceding claim, wherein the guide passage is provided within the catheter wall.

6. An apparatus as claimed in any preceding claim, wherein the guide member incorporates leads and/or a driveshaft for the imaging device.

7. An apparatus as claimed in any preceding claim, wherein the imaging device is mounted to be reciprocated within the guide member.

8. An apparatus as claimed in claim 7, wherein the imaging device is rotatably mounted within the guide member.

9. An apparatus as claimed in any preceding claim, wherein the imaging device is connected to a computer for producing an image of the tissue immediately around and ahead of the catheter tip.

10. An apparatus as claimed in claim 9, wherein the computer is programmed to provide a three dimensional toroidal or substantially toroidal image made up from a series of two dimensional cross sectional views provided by the imaging device at intervals around the circumference of the catheter.

11. An apparatus as claimed in claim 9, wherein the computer is programmed to provide a two dimensional projection of said three dimensional image.

12. An apparatus as claimed in any preceding claim, wherein the catheter is adapted to be advanced over a cardiac lead (4) to assist removal of the lead.

13. An apparatus as claimed in any preceding claim, wherein one or more further tubes or catheters are provided concentrically outside of or inside of the catheter to assist lead removal

14. An apparatus as claimed in any preceding claim, further comprising additional lumens or other tubular members within or adjacent to the catheter through which guide wires, stents, and/or balloons may be introduced for other therapeutic modalities, such as for the purpose of evaluating and/or treating coronary artery narrowings or occlusions.

15. An apparatus as claimed in claim 14, wherein one or more additional lumens or other tubular members may be provided for the passage of one or more of biopsy forceps, electrode catheters, including mapping and radiofrequency ablation catheters, or guide wires or any other device for use in other diagnostic and/or therapeutic procedure.

16. An apparatus as claimed is any preceding claim wherein the guide passage includes a helical portion connecting the circumferential portion (x) to the remainder of the guide passage.

17. An apparatus as claimed in an preceding claim wherein the tip of the catheter is a distal end of a distal portion

## Patentansprüche

1. Gerät zur Durchführung von Diagnose- und/oder Therapieverfahren, wobei das Gerät einen hohlen, röhrenförmigen Katheter (10) zur Einführung in das venöse oder arterielle System (2) oder eine andere Körperhöhle eines Patienten umfasst, wobei der Katheter aufweist: eine Spitze (12), eine mit der Spitze des Katheters in Verbindung stehende Bilderzeugungsvorrichtung, wobei die Bilderzeugungsvorrichtung so angepasst ist, dass sie ein Bild des Gewebes vor und seitlich der Katheterspitze bereitstellt, wobei die Bilderzeugungsvorrichtung an einem distalen Ende eines länglichen Führungselements vorgesehen ist, das in einem Führungsdurchlass (14) geführt wird, um in dem Führungsdurchlass axial beweglich zu sein, **dadurch gekennzeichnet, dass** sich ein Abschnitt (x) des Führungsdurchlasses an der oder benachbart zur Spitze des Katheters in einer Umfangskonfiguration um einen distalen Bereich des Katheters rechtwinklig zur Hauptachse des Katheters erstreckt, wodurch die Bilderzeugungsvorrichtung zumindest teilweise in Umfangsrichtung um einen Teil der Spitze des Katheters hin- und herbewegt werden kann, um ein ringförmiges oder im Wesentlichen ringförmiges Bild (N) des Gewebes unmittelbar um die und vor der Katheterspitze zu erzeugen.

2. Gerät nach Anspruch 1, wobei die Bilderzeugungsvorrichtung so angepasst ist, dass sie der Katheterspitze benachbartes Gewebe vorwärts und seitlich sichtbar macht.

3. Gerät nach Anspruch 1 oder Anspruch 2, wobei die Bilderzeugungsvorrichtung so angepasst ist, dass sie zwischen Muskel, Knochen und Blut unterscheidet und so feststellt, ob der Katheter in Bereiche vorgeschoben wird, in denen Gewebe, z. B. die Wände von Venen und Arterien, beschädigt werden können.

4. Gerät nach einem vorangehenden Anspruch, wobei die Bilderzeugungsvorrichtung eine Ultraschall-Bilderzeugungsvorrichtung umfasst, die einen oder mehrere Ultraschallwandler umfasst.

5. Gerät nach einem vorangehenden Anspruch, wobei der Führungsdurchlass in der Katheterwand vorgesehen ist.

6. Gerät nach einem vorangehenden Anspruch, wobei das Führungselement Leitungen und/oder eine Antriebswelle für die Bilderzeugungsvorrichtung einschließt.

7. Gerät nach einem vorangehenden Anspruch, wobei die Bilderzeugungsvorrichtung so angebracht ist, dass sie in dem Führungselement hin- und herbewegt wird.

8. Gerät nach Anspruch 7, wobei die Bilderzeugungsvorrichtung drehbar in dem Führungselement angebracht ist.

9. Gerät nach einem vorangehenden Anspruch, wobei die Bilderzeugungsvorrichtung zwecks Erzeugung eines Bildes des Gewebes unmittelbar um die und vor der Katheterspitze mit einem Computer verbunden ist.

10. Gerät nach Anspruch 9, wobei der Computer so programmiert ist, dass er ein dreidimensionales ringförmiges oder im Wesentlichen ringförmiges Bild bereitstellt, das aus einer Reihe zweidimensionaler Querschnittsansichten erstellt wird, die von der Bilderzeugungsvorrichtung in Abständen um den Umfang des Katheters herum bereitgestellt werden.

11. Gerät nach Anspruch 9, wobei der Computer so programmiert ist, dass er eine zweidimensionale Projektion des dreidimensionalen Bildes bereitstellt.

12. Gerät nach einem vorangehenden Anspruch, wobei der Katheter so angepasst ist, dass er über eine Herzelektrode (4) vorgeschoben wird, um die Entfernung der Elektrode zu unterstützen.

13. Gerät nach einem vorangehenden Anspruch, wobei ein(e) oder mehrere weitere(r) Röhre(n) oder Katheter konzentrisch außerhalb oder innerhalb des Katheters vorgesehen sind, um die Elektrodenentfernung zu unterstützen.

14. Gerät nach einem vorangehenden Anspruch, das weiterhin zusätzliche Lumen oder andere röhrenförmige Elemente in dem oder benachbart zum Katheter umfasst, durch die Führungsdrähte, Stents und/oder Ballons für andere Therapiemodalitäten eingeführt werden können, z. B. zwecks der Beurteilung und/oder Behandlung von Verengungen oder Verschlüssen der Koronararterien.

15. Gerät nach Anspruch 14, wobei ein oder mehrere zusätzliche(s) Lumen oder andere(s) röhrenförmige(s) Element(e) vorgesehen sein können, zum Durchführen eines oder mehrerer der folgenden: Biopsiezangen, Elektrodenkatheter, einschließlich Mapping- und Hochfrequenz-Ablationskatheter, oder Führungsdrähte oder eine andere Vorrichtung zur Verwendung bei einem anderen Diagnose- und/oder Therapieverfahren.

16. Gerät nach einem vorangehenden Anspruch, wobei der Führungsdurchlass einen schraubenförmigen Abschnitt umfasst, der den Umfangsabschnitt (x) mit dem Rest des Führungsdurchlasses verbindet.

17. Gerät nach einem vorangehenden Anspruch, wobei sich die Spitze des Katheters an einem distalen Ende eines distalen Abschnitts befindet.

## Revendications

1. Appareil permettant de réaliser des procédures diagnostiques et/ou thérapeutiques, ledit appareil comportant un cathéter (10) tubulaire creux destiné à être introduit dans le système (2) veineux ou artériel ou dans une autre cavité corporelle d'un patient, ledit cathéter étant doté d'une pointe (12), un dispositif d'imagerie étant associé à la pointe du cathéter, ledit dispositif d'imagerie étant conçu pour fournir une image des tissus devant la pointe du cathéter et latéralement par rapport celle-ci, dans lequel le dispositif d'imagerie est fourni au niveau d'une extrémité distale d'un élément de guidage allongé guidé dans un passage (14) de guidage afin d'être déplacé en translation axiale dans le passage de guidage, **caractérisé en ce qu'**une portion (x) du passage de guidage au niveau ou à côté de la pointe du cathéter s'étend dans une configuration circonférentielle autour d'une région distale du cathéter perpendiculairement à l'axe principal du cathéter, moyennant quoi le dispositif d'imagerie peut au moins partiellement être animé d'un mouvement de va-et-vient circonférentiel autour d'une partie de la pointe du cathéter pour produire une image (N) toroïdale ou sensiblement toroïdale des tissus immédiatement autour de la pointe de cathéter et devant celle-ci.

2. Appareil selon la revendication 1, dans lequel le dispositif d'imagerie est conçu pour fournir une visualisation vers l'avant et latérale des tissus adjacents à la pointe du cathéter.

3. Appareil selon la revendication 1 ou 2, dans lequel ledit dispositif d'imagerie est conçu pour pouvoir distinguer les muscles, les os et le sang et par conséquent déterminer si le cathéter est avancé dans des zones pouvant comporter des lésions tissulaires, telles que les parois veineuses ou artérielles.

4. Appareil selon l'une quelconque des revendications précédentes, dans lequel ledit dispositif d'imagerie comporte un dispositif d'imagerie à ultrasons comportant un ou plusieurs transducteurs à ultrasons.

5. Appareil selon selon l'une quelconque des revendications précédentes, dans lequel le passage de guidage est fourni dans la paroi de cathéter.

6. Appareil selon l'une quelconque des revendications précédentes, dans lequel l'élément de guidage incorpore des conducteurs et/ou un arbre d'entraînement pour le dispositif d'imagerie.

7. Appareil selon l'une quelconque des revendications précédentes, dans lequel le dispositif d'imagerie est monté pour assurer un mouvement de va-et-vient dans l'élément de guidage.

8. Appareil selon la revendication 7, dans lequel le dispositif d'imagerie est monté rotatif dans l'élément de guidage.

9. Appareil selon l'une quelconque des revendications précédentes, dans lequel le dispositif d'imagerie est relié à un ordinateur destiné à produire une image des tissus immédiatement autour de la pointe de cathéter et devant celle-ci.

10. Appareil selon la revendication 9, dans lequel l'ordinateur est programmé de manière à fournir une image toroïdale ou sensiblement toroïdale tridimensionnelle constituée d'une série de vues en coupe transversale bidimensionnelles fournies par le dispositif d'imagerie à certains intervalles autour de la circonférence du cathéter.

11. Appareil selon la revendication 9, dans lequel l'ordinateur est programmé de manière à fournir une projection bidimensionnelle de ladite image tridimensionnelle.

12. Appareil selon l'une quelconque des revendications précédentes, dans lequel le cathéter est conçu pour être avancé sur un conducteur (4) cardiaque afin d'aider au retrait du conducteur.

13. Appareil selon l'une quelconque des revendications précédentes, dans lequel un ou plusieurs tubes ou cathéters sont fournis disposés de manière concentrique à l'extérieur ou à l'intérieur du cathéter afin d'aider au retrait du conducteur.

14. Appareil selon l'une quelconque des revendications précédentes, comportant en outre une lumière supplémentaire ou d'autres éléments tubulaires à l'intérieur ou à côté du cathéter à travers lesquels des fils de guidage, des tuteurs, et/ou des ballonnets peuvent être introduits pour d'autres modalités thérapeutiques, telles que pour l'évaluation et/ou le traitement de rétrécissements ou d'occlusions d'artères coronariennes.

15. Appareil selon la revendication 14, dans lequel une ou plusieurs lumières supplémentaires ou d'autres élément tubulaires peuvent être fournis pour le passage d'une ou plusieurs pinces à biopsie, cathéters à électrode, comprenant des cathéters de cartographie et d'ablation haute fréquence, ou fils de guidage ou autres dispositifs destinés à être utilisés dans d'autres procédures diagnostiques et/ou thérapeutiques.

16. Appareil selon l'une quelconque des revendications précédentes dans lequel le passage de guidage comprend une portion hélicoïdale reliant la portion (x) circonférentielle au reste du passage de guidage.

17. Appareil selon l'une quelconque des revendications précédentes dans lequel la pointe du cathéter est une extrémité distale d'une portion distale.
